# EUROPEAN PATENT APPLICATION

(11) **EP 4 137 557 A1**
(43) Date of publication of application: **22.02.2023**
(21) Application number: 21382765.2
(22) Date of filing: 18.08.2021
(51) Int. Cl.: C12M 3/00, C12M 1/00

(54) **DEVICE FOR PRODUCING BIOMASS AND ITS DERIVATIVES**

(71) Applicant: Horacio, Acerbo Claudio, 1061 Ciudad de Buenos Aires (AR); Blasco, Martín, 1426 Ciudad de Buenos Aires (ES); Gago, Lucas Martín, 29651 Málaga (ES)
(72) Inventor: Horacio, Acerbo Claudio, 1061 Ciudad de Buenos Aires (AR); Blasco, Martín, 1426 Ciudad de Buenos Aires (ES); Gago, Lucas Martín, 29651 Málaga (ES)
(74) Representative: Isern Patentes y Marcas S.L.

(57) **Abstract**

A device for the decentralized and continuous production of biomass at home, which comprises: a culture container for continuous processes, in batch and in a fed batch connected to a system that provides gaseous components; and all the supplied components are sterilized by a filter; a solids dispenser that is connected to a solids reservoir, and which can quantify the dispensed solids; a dissolving-unit that is connected to the solids dispenser, to a liquids metering pump, and to a purified water generation system, and where the dissolving-unit exit is connected to the culture tank by the culture medium filters; and the latter communicate the fluids with the dissolving-unit and the culture tank; a device that contains and dispenses the inoculum that initiates the culture, and which is connected to the culture tank; a temperature-controlled rotary drum system is connected to the culture tank to separate the biomass, with a drain of liquids; a sterilizer is connected to the culture tank and the associated filters; a microprocessor with instructions to control the variables of the process, which is connected to a user interface; and connections to a water source, electric power source, drain of liquids, and to Internet and servers with Al. Procedure for the continuous production at home of biomass using said device.

## Description

### Area of Invention

The invention herein belongs to the area of processes and appliances used for the production of biomass and other biotechnological products. It is particularly related to the production of components that are of interest for the daily development of activities of human or animal nutrition. And more particularly, it is related to a process and device that allows the production of edible components through bioprocesses.

### Background

Microorganisms are essential components of the terrestrial ecosystem, and they have a wide diversity that includes the entire already known biological niche. The exploitation of microorganisms and their active derivatives (for example, enzymes) by human beings has gone through all the stages of human development, and in some cases it was unnoticed by the users. By virtue of the development in the scientific and technological knowledge, this area started to be controlled by developing a system to increase the productivity of the biotechnological processes. However, said optimized processes were rarely adopted for their use at a family level, and the major part of this exploitation is done in industries, where full size equipment of complex operation is used.

Although small-size fermenters are already known, they have not been intended to operate at a home environment with a little, or even no professional control. Additionally, they do not have systems that allow the remote control of the microbiological status of the culture. This control is done by using a system that receives images, or by analyzing the parameters of the process, so that alarms can be set to alert whenever there is a possible contamination with other microorganisms. Applying Artificial Intelligence (Al) and Internet-of-Things (IOT) makes it easier to implement this type of technologies to control complex processes. Additionally, the use of encryption systems such as block-chain would be useful for protecting the data related to the process.

For the exploitation of microorganisms at a home level or on a small scale it is necessary to have a device of small dimensions, where the culture medium must be prepared within the line of production based on solid raw material and liquid components, such as water, which is added in great amounts, and other trace elements that are added in small amounts. Another important fact is the need of devices capable of gathering information for the quality control of the process.

The optimization of the culture media is a fundamental part of those operations, which are intended to make this successful equipment real, because it allows the reduction of the containers scales, but mainly because it reduces the production cost when using this equipment at home or on a small scale.

### Summary of the Invention

Due to the above, it is an object of this invention to provide a device for the decentralized (or small-scale) home production of biotechnological derivatives (for example, biomass or derivatives of biological processes) that includes:
A reservoir of culture, which allows the circulation of the medium, and which has a system that provides fluids (for example, air, oxygen or liquids);
A dispenser of solids that is connected to a reservoir of solids, and which can quantify the dispensed solids;
A dissolving-unit which is connected to the solids dispenser, to a liquids metering pump and to a purified water generation system, and where the dissolving-unit exit is connected to the culture tank by the culture medium filters. The latter communicate the fluids with the dissolving-unit and the culture tank.
A device that contains and dispenses the inoculum that initiates the culture, and which is connected to the culture tank;
A separating system by filtration (for example, a temperature-controlled rotary drum) is connected to the culture tank to separate the solid and liquid products.
A sterilizing system that is connected to the culture reservoir and the associated filters;
A control programming system connected to a user interface in order to control the variables of the process; and
Connections to a water source, electric power source, drain of liquid, and optionally, to Internet.

Preferably, the device that contains and dispenses the inoculum that initiates the culture includes a cassette that has multiple initiators of the culture, even enough to cover the lifetime of the equipment.

It is also preferably if the sterilizer is a water vapor generator or a vapor generator of H₂O₂, and a coupled reservoir.

It is even more preferably if the microprocessor includes Al instructions that control and improve every aspect of the production procedure.

It is still more preferably if any source of electric power provides the adequate power to the equipment (for example but not limited to, 220 V - 50 Hz, 20 A or 110 V-60 Hz 40 A).

It is another object of this invention to provide a procedure for the continuous home production of biomass by using the device from claim 1, which consists of:
a) Connecting the device to a water source, a power electric source, a drain of liquids, Internet and to the server for the remote control of the process, where AI is implemented;
b) Activating the filters of the culture medium, water and air;
c) Placing a cassette with a spore of inoculum in its receptacle;
d) Loading the solids for preparing the culture medium in the solids reservoir;
e) Placing the container with the liquid components of the process and connecting it to the system for preparing the culture medium;
f) Optionally, adding a sterilizing agent;
g) Switching on the equipment and selecting the start-process option;
h) Cultivating the microorganism until the content of the solid tank is over, or alternatively until the microorganism has visible variations in the expected result; and
i) Separating the biomass or the components of interest in a continuous form.

In a preferred form, the cassette has enough inoculum seeds either for the lifetime of the equipment, or until greater maintenance services are done.

In a preferred form as well, the inoculum is a microorganism, which has received a treatment that allows its maintenance at ambient temperature without significantly reducing its viability (for example, freeze-drying).

In an even preferred form, the solids reservoir comprises 5 - 100 kg of solids to prepare the culture medium.

In a more preferred form, the sterilizing agent is pressurized water vapor or H₂O₂ vapor.

### Brief Description of the Drawings

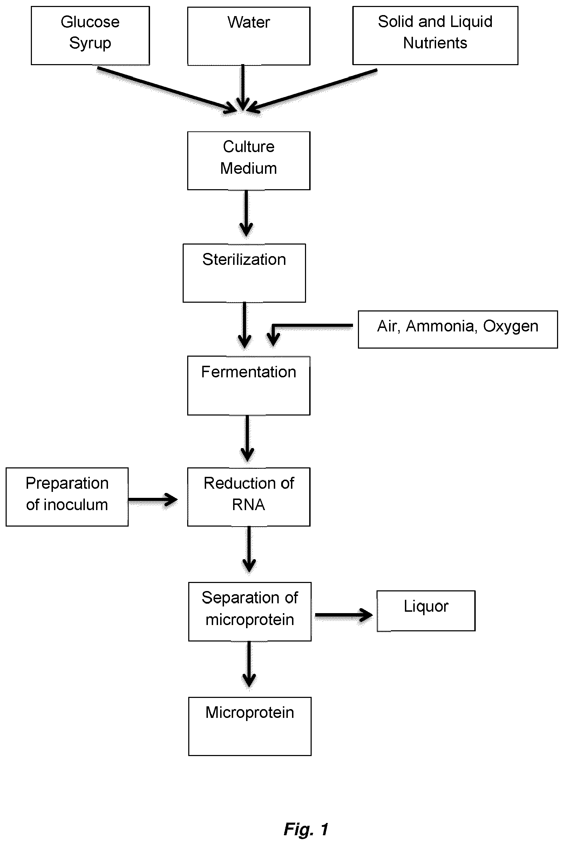

Figure 1 shows a logical diagram of the production procedure of biomass on a home scale.

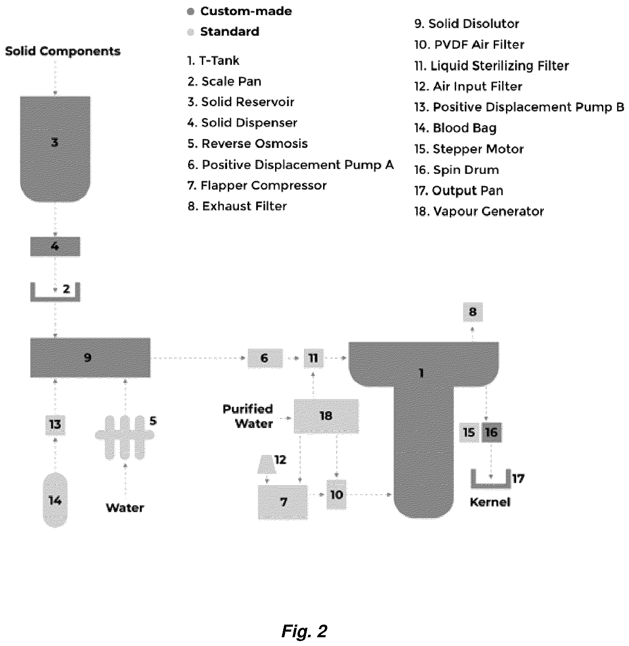

Figure 2 shows a schematic preferred embodiment of the device according to this invention, and its components.

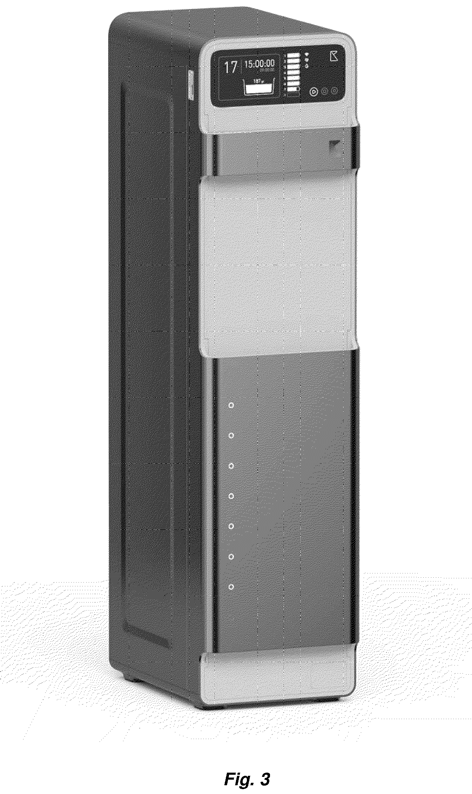

Figure 3 shows the exterior of a preferred form of the device for the production of biomass at home of this invention.

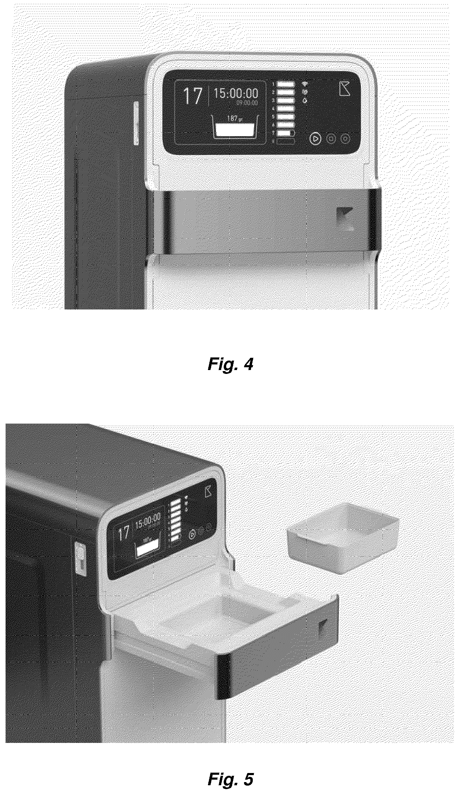

Figure 4 shows the upper part with the user interface of the device for the production of biomass at home from Figure 3.
Figure 5 shows the pan and pan-holder, where the culture that is in the upper part of the device for the production of biomass at home from Figure 3 is made.

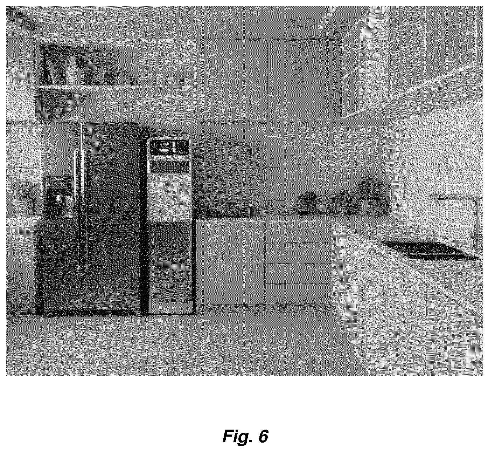

Figure 6 shows the device for the production of biomasses or their derivatives at home from Figure 3, which is preferably installed in the kitchen area of a house.

### Detailed Description of the Invention

The products obtained from the device according to this invention, have the aim of decentralizing the production and providing access to all human beings, which is a completely possible and **original** fact.

The electric power system, Internet and block-chain are only some examples of how the decentralization can improve the way in which human beings live, produce and interact.

In the same way that Bitcoin was the first expression of block-chain technology, the decentralization of the production of biomass or other products of human interest is just the first step towards a future, where all will be able to produce their own food or raw materials in a sustainable manner.

Producing biomass with a system of multiple little centers is the best way to take the product everywhere. Therefore, the need for transport would be reduced, and packaging would not be necessary. Furthermore, its production is sustainable, because it reduces the footprints of water and carbon.

Therefore, it is an object of this invention to achieve the production of biomass and its derivatives to reach most of the inhabitants of this planet, if not all of them, with a reduced environmental impact.

The device of this invention will allow any person to be able to prepare these productions of microorganisms in any place, such as, a family house, a school, a building, or a neighborhood.

Moreover, it is possible to continuously improve the process of production by applying Artificial Intelligence (Al). Actually, it is by means of this technology that thousands of conditions can be analyzed to find the suitable combination for producing in a more economical and efficient manner.

With AI controlling every aspect of the devices and learning how the process of production occurs, it will be able to optimize the procedure for the immediate future.

The conditions to be fulfilled by the device of this invention are in a broad sense, as follows:
- System of Continuous Process.
- Capacity: 1/15 liters.
- Duration of the process set by the client or until the components are ran out, or if the product is affected.
- Optimized culture medium.
- Filtration

### Description of the Process

### Outline of the Process

The production process corresponds to a typical biotechnological process for producing biomass. The steps to follow towards the production process are outlined herein. When the equipment is used at home some things change, for example, all those components that in the industrial version can be used in liquid form have to be solid. For example, a culture system that may require fluid milk would be replaced by powdered milk, the glucose syrup would be replaced by solid glucose, molasses would be replaced by saccharose, etc. The process for sterilizing the culture medium is done through filters or other sterilization system capable of processing liquids within the line of the production process. The complex gaseous additives are replaced by solid versions, such as the use of ammonia is replaced by ammonium salts or other component that adds nitrogen to the system. Among the gaseous additives, it is possible to find the air or gaseous fractions that are rich in oxygen or nitrogen deriving from the separation processes of the air. The preparation of inoculum or reaction agent is replaced by adding a tab or any other form characterized by its long-term stability (ideally for years).

The production is continuous until any of the supplies is ran out or if the product is deviated. In those cases, where treatments for reducing components by enzymatic processes are necessary, said treatments will be held in a reaction chamber that is separated from the culture reservoir. These treatments can be either thermic (reaction with components and catalysts from the production itself) or enzymatic with external additives (for example, reducing the content of RNA by thermic treatment for the first case; oligosaccharide formation resulting from sugar catalyzed by ligases. The reaction products can be separated in the liquid-solid separation system, and the liquid can be concentrated for other uses (for example, the products of RNA degradation could be concentrated and stored for their later use in order to improve the taste). When the composition of liquid effluents allows it, part of the water from the composition can be recycled reducing the need of water in the process. This process would be held internally by using the system of reverse osmosis.

### Operations for installing the equipment

The following operations must be complied for the correct functioning of the equipment:
1. Connect the equipment to a water source.
2. Connect the equipment to an electric power source.
3. Connect the equipment to a drain of liquids.
4. Connect the equipment to Internet.
5. Place the filters of culture medium, water and air in their position.
6. Place the seed cassette. Preferentially, the cassette is composed of enough seeds for the lifetime of the equipment.

### Preliminary operations for starting the process

The following operations must be complied every time the production starts:
1. Load the solids for preparing the culture medium (5 - 100 kg) in the solids reservoir.
2. Place the container with liquid components of the process (bag with liquid and hose), and connect it to the system for preparing the medium (dissolving-unit). In a preferred embodiment, this component could be loaded per a great number of processes (5-10 processes).
3. Add the sterilizing agent (if it corresponds considering that the H₂O₂ mode was selected).
4. Switch on the equipment.
5. Select the start-process option.

### Sterilization

Before beginning with a new production cycle the equipment must be sterilized. The proceeding can be done by using physical or chemical agents (for example, temperature, vapor, H₂O₂ or any other equivalent).

The aim of this proceeding is:
1. To guarantee the microbiological safety (to prevent a contaminating bacteria, fungus or yeast from affecting the product or the user's health).
2. To guarantee no living components are swept along from one process to another.

If vapor is used as a sterilizer (high temperature, 121 °C and 1.2 bar of pressure), the equipment must have a vapor generator with sufficient capacity for the treatment of the tank and sterilizable peripherals (air filters, medium, etc.). The designs of the reservoir and filters must take into account the passage of the vapor for a fraction of the sterilization time, which would be ideally 10 minutes. The T-tank should be treated at 121°C for 20 minutes.

If H₂O₂ is used, the energy consumption is reduced, and the process is carried out at a normal pressure, but it will be necessary to load an extra supply to the equipment (H₂O₂). The process consists of evaporating H₂O₂ and making the vapor travel through all the system. This process must be validated because in general terms it is not used for this purpose. It may necessary to clean the system with water in order to prevent this from affecting the final product.

### Preparing the culture medium

This operation will be done several times during the process in order to supply the necessary culture medium for the correct functioning of the equipment. The volume of preparation of each day strictly depends on the volume of the T-tank. The relationship is the volume of the T-tank multiplied for approximately 4.6, which is equal to the quantity of the culture medium per day.

If the volume of the T-tank is 15 liters, the quantity to be prepared per day will be approximately 70 liters. The velocity of the production of the medium must be enough to supply the medium to the T-tank continuously. That is to say, the consumption will be half per 0.0475 L.min⁻¹.

The system for preparing the culture medium consists of:
1. The solids dispenser, which is a unit capable of defining a quantity of solids and adding them to a preparation tank, where they will be mixed with water and liquid nutrients. The production of batches of culture medium is done in accordance with the capacity of the medium preparation tank and with the capacity to dispense from the liquids dispenser. The calculations can be done, for example, by using the data from Table 1 set forth below.
2. A tank for dissolving.
3. A liquids metering pump.
4. A system for producing purified water.

Preparing the culture medium consists of adding an equal quantity of solids to the dissolution system, adding the necessary quantity of traces and enough water, which is water produced by an accessory osmosis equipment. Once it is mixed, the pumping is done in a controlled manner, for example, through a metering pump capable of providing enough pressure to the T-tank through a sterilizing filter of the liquid culture medium at a rate of 70 liters per day.

In case the dissolving-unit is capable of preparing 2.8 liters, the process can start after two preparations of medium. The preparation should not take longer than 3 minutes per batch. It is considered that the preparation of a quantity to cover the first set of two bubblers of the T-tank is enough. In a preferred embodiment it would be about 5 liters.

Once this situation is reached, the regulation of the process temperature must start in order to achieve the ideal value (28 - 30°C).

### Inoculation of the T-tank

The inoculation of the T-tank is done every time a new process of 22-day production starts.

Once the T-tank and the accessories are sterilized, and after producing approximately 5 liters of culture medium, the inoculation starts.

For the inoculation of the fermenter, in a preferred embodiment, it could be an option to use a cartridge with tabs of the freeze-dried microorganism. The cartridge must be designed in a way to keep the entirety of the tabs, and to make sure the tab falls in the culture medium inside the T-reactor in order to start the process. This operation must be done without letting external material in.

### Beginning of the culture

The entrance of air, which must be sterile and filtered, begins at a rate of 30 liters per min⁻¹ to maintain the necessary conditions of oxygen for the culture. After 24 hours of having started, the feeding initiates, and it will continue according to the expected volume, preferably 70 liters per day⁻¹. The production of the culture medium will be according to the production system, completing the volume of the reactor and producing the controlled overflow thereof once the 15 liters of volume are reached. It is estimated to last 3.5 hours after the 24 hours of start.

### Complete culture (20 days)

In the T-tank, the culture medium is mixed with the culture, where the microorganism of interest is multiplying by constant airing, temperature, and entering volume of medium. As new culture medium enters the culture with biomass drains from the T-tank to the system of biomass harvesting.

### Continuous harvesting

As the culture, which is composed of exhausted culture medium and biomass, is drained from the T-tank, it is rendered inactive by temperature in a system of rotary drum (small system).

The outlet of material from the equipment must be designed in a way that the solids of the process do not deposit in the intermediate stages before reaching the rotary drum for the filtration. It may be possible to design a screw system to secure the cleaning of the outlet system of the grown culture.

In the harvest chamber, the culture medium falls on a rotary drum with a 10-micron filter, where vacuum is created, and the liquid travels to the effluents drain, while the solid is removed by a scraping system that directs it to the deposit pan.

The drum is at a controlled temperature (for example, at about 64°C to produce the reduction of RNA). The time spent on the surface of the drum is regulated by the angular velocity of the drum (for example, 0.1 min-1) and the position of the system for removing solids.

Once the biomass is removed from the drum surface by a blade, the biomass falls in the deposit pan in such a way that the solid is kept at a low temperature until being picked by the user.

### End of the culture (for example, +20 days for exhaustion of the medium or instability of the microorganism)

Once the time limit of the process is reached, there will be a stop of the process by which the maximum volume of water that the osmosis team is capable to endure will be injected to the system. This enables the cleaning of the system leaving it ready for its sterilization and next process.

### Requirements

### - Air of the process

The air is pushed forward by a "flapper-type" compression unit or any other type of compression unit suitable for the required volume. It has to be considered that the 30 L min⁻¹ must be able to be aired out to prevent an increase in the pressure.

### - Temperature control

The T-reactor must keep the temperature controlled at about 30 °C in a range capable for the equipment to operate. It could be possibly done through Peltier cells.

It is possible to isolate the tank to prevent a thermal unbalance in cases of high or low temperature.

### - Water of the process

It is water obtained from a reverse osmosis process, which is free of chlorine, and it is treated according to the requirements included in Table 1 above. - Generation of vapor

The sterilizing system requires vapor at a rate of 20 kg of water per hour for 30 minutes.

### - Evaporation with peroxide (it is an alternative to the generation of vapor)

As an alternative to vapor, it is possible to produce vapors of hydrogen peroxide for sterilizing at a normal pressure. In said case, it is provided a vapor generator of low pressure like the ones used in clothes dryers but with improvements, in order to avoid the corrosion of certain parts that may be affected by the peroxide.

### Components of the Device

### - T-tank

It has a proportional ascending branch with respect to the descending branch, and an enlargement in the top for controlling the foam.

It includes air-insufflation systems, and mixing capacity upon a complex rheology of the culture. It has an adequate design to support the mycelial growth and to certify the effective operation of the equipment.

### - Dispenser of solids

There is a correct connection and flow of solids from the solids reservoir to the solids dispenser.

There is an adequate procedure for dispensing and quantifying solids.

There is no contamination under the usual conditions of the process. Barriers are defined in order to have an aseptic operation.

### - Dissolving-unit

Minimum mixing times are defined.

There is no contamination under the usual conditions of the process. Barriers are defined in order to have an aseptic operation.

### - Filters of the culture medium

There is no microbiological accumulation during the time of operation. There is a need for a regular treatment to avoid the increase of mass of microorganisms.

### - Air filters

There are two filters, one is for the air to enter the *"flapper"* in order to avoid deterioration, and the other is the filter for sterilizing.

There is no microbiological accumulation during the operation time. There is a need for a regular treatment to avoid the increase of mass of microorganisms.

### - Sterilization within the line of production of the culture medium

Preparing the culture medium in a continuous form demands to the sterilizing filters to meet a specific condition. Considering that the filters have absorbed the culture medium, any microorganism capable of growing in said medium will end up saturating the filter within a significant time.

In this context, the filtration system must be capable of facing this situation.

A way of facing this situation is the frequent sterilization of the filter to reduce the bacterial load (for example, with heat).

This can be done in different ways depending on the elements of filtration that are available in the market.

A filter with an associated device capable of eliminating contaminant microorganisms could be:
- An internal heating system that can be activated regularly to kill microorganisms.
- An ultrasound system to lyse microorganisms.
- An ultraviolet light system to light up the system at regular intervals to kill microorganisms (without culture medium).
- An electrode system that applies oscillating potential differences with deleterious characteristics for the microorganisms.
- A system of reversed flow that detaches and washes (backwashing) the accumulated microorganisms.
- There could be some kind of bacteriostatic agent in the material of the filter to prevent the microorganisms that fall in the filter to reproduce.
- A system that sterilizes the filter at regular intervals through vapor of water or H₂O₂.
- To use sterile solids and sterile water to prepare the culture medium, reducing the possibility to contaminate the medium and the filters.
- A tangential filtration system that in the event of growth can recirculate, and possibly dispose of the liquid to reduce the accumulation of microorganisms in the filtering surface.

## Claims

1. A device for the decentralized and continuous production of biomass at home, which comprises:
A culture container that is connected to a culture medium provision or reaction, a system that supplies air, oxygen or other gases, and a sterilizing filter coupled in series;
A solids dispenser that is connected to a solids reservoir and which can quantify the dispensed solids;
A continuous or semi-continuous dissolving-unit that is connected to the solids dispenser, to a liquids metering pump, and to a purified water generation system, and where the dissolving-unit exit is connected to the culture tank by the culture medium filters. The latter communicate the fluids with the dissolving-unit and the culture tank.
A device that contains and dispenses the inoculum that initiates the culture, and which is connected to the culture tank;
A temperature-controlled separating system is connected to the culture tank to separate the biomass or the reaction products, with a drain of liquids.
A sterilizing system inside is connected to the culture tank and the associated filters;
A microprocessor system to control the process is connected to a user interface; and
Connections to a water source, electric power source, drain of liquids, to Internet, and to servers.

2. The device from claim 1, wherein the device that contains and dispenses the inoculum that initiates the culture includes a cassette that has sufficient seeds for the equipment to function in a continuous form during multiple processes, ideally during the whole lifetime of the equipment.

3. The device from claim 1, wherein the sterilizer is a water vapor generator or a vapor generator of H₂O₂, and a coupled reservoir.

4. The device from claim 1, wherein the microprocessor includes AI instructions that control and improve every aspect of the production procedure.

5. The device from claim 1, wherein the source of energy power is electric.

6. A procedure for the continuous home production of biomass by using the device from claim 1, which consists of:
a) Connecting the device to a water source, a power electric source, a drain of liquids, and optionally, to Internet;
b) Activating the filters of the culture medium, water and air;
c) Placing a cassette with a seed of inoculum in its receptacle;
d) Loading the solids for preparing the culture medium in the solids reservoir;
e) Placing the container with the liquid components of the process and connecting it to the system for preparing the culture medium;
f) Optionally, adding a sterilizing agent;
g) Switching on the equipment and selecting the start-process option;
h) Cultivating the microorganism in a continuous form until the reserves of medium are over, or upon genetic instability of the microorganism; and
i) Stopping the process, and separating the resulting biomass.

7. The procedure from claim 6, wherein the cassette has sufficient units of inoculum for multiple continued processes, without having to add more.

8. The procedure from claims 6 or 7, wherein the inoculum is freeze-dried in order to have a long-time stability for initiating each culture.

9. The procedure from claim 6, wherein the solids reservoir comprises 5 - 100 kg of solids to prepare the culture medium.

10. The procedure from claim 6, wherein the sterilizing agent is water vapor or H₂O₂ vapor.
